# EUROPÄISCHE PATENTSCHRIFT

(11) **EP 0 561 307 B1**
(45) Veröffentlichungstag und Bekanntmachung des Hinweises auf die Patenterteilung: **06.08.1997**
(21) Anmeldenummer: 93104101.6
(22) Anmeldetag: 13.03.1993
(51) Int. Cl.: A61K 31/045

(54) **Antirauchmittel**
Smoking cessation agent
Agent pour la cessation de fumer

(30) Priorität: 14.03.1992 DE 4208211
(43) Veröffentlichungstag der Anmeldung: 22.09.1993
(73) Patentinhaber: Hadek, Karel, D-82024 Taufkirchen (DE)
(72) Erfinder: Hadek, Karel, D-82024 Taufkirchen (DE)
(74) Vertreter: von Kirschbaum, Albrecht, Dipl.-Ing.

(56) Entgegenhaltungen:
- DRUG AND ALCOHOL DEPENDENCE, Bd. 26, 1990, Seiten 155-160; E.D. LEVIN ET AL.: 'The use of flavor in cigarette substitute'

## Beschreibung

Die Erfindung betrifft die Verwendung eines Gemisches zur Herstellung eines Arzneimittels.

Nachdem in der letzten Zeit immer häufiger von Rauchern akzeptiert wird, daß die nachteiligen Wirkungen des Tabakkonsumes sämtliche positiven Wirkungen des Tabakkonsumes überwiegen, versuchen immer mehr Raucher das Rauchen aufzugeben.

Dabei stoßen die Raucher jedoch auf das Problem, daß sich der Körper an die Nikotinzufuhr durch das Rauchen gewöhnt hat. Die Wirkungen des Nikotins sind hinlänglich bekannt, so z.B., daß von dem Nikotin eine beruhigende Wirkung ausgeht. Mit anderen Worten, insofern der Raucher unter Nikotinentzug steht, wird er im Laufe der Zeit immer unruhiger und nervöser, ja sogar aggressiv und greift schließlich doch wieder zur Zigarette.

Es sind verschiedene Methoden bekannt geworden, um Rauchern das Einstellen des Rauchens zu erleichtern. Dabei wird den Rauchern z.B. in Kaugummis oder über Pflaster Nikotin verabreicht, das durch die Mundschleimhäute oder über die Haut in den Blutkreislauf gelangt und dort seine Wirkung tut, ohne daß der Raucher zu diesem Zweck einen rauchbaren Artikel bzw. eine Zigarette rauchen müßte.

Allerdings haben diese Entwöhnungsmittel den Nachteil, daß es sich bei ihnen im Prinzip um keine Entwöhnungsmittel handelt, sondern nur um Suchtersatzmittel , die bewirken, daß dem Raucher weiterhin Nikotin zugeführt wird, jedoch ohne ihn zusätzlich durch die in den Zigaretten enthaltenen Kondensate zu belasten. Stellt der Raucher den Konsum von derartigen Nikotin enthaltenden Kaugummis oder von derartigen Nikotinpflastern ein, so treten wieder die Entzugserscheinungen auf und der Raucher ist wieder in Gefahr zur Zigarette zu greifen.

Andere Methoden zur Erleichterung der Entwöhnung basieren auf Akkupunktur bzw. auf Akkupressur. Dabei muß der betreffende Raucher sehr häufig und teilweise über Monate hinweg und länger sachgerecht behandelt werden, was mit einem erheblichen zeitlichen Aufwand verbunden ist, was sehr häufig zum Abbruch der Behandlung führt.

Gemäß Drug and Alcohol Dependence, 1990, Bd.28, S.155 bis 160 kann das Verlangen von Zigarettenrauchern nach "richtigen" Zigaretten reduziert werden, wenn ihnen Placebo-Zigaretten (ohne Tabak) mit Mentholaroma angeboten werden. Das Mentholaroma wird durch eine in der Placebo-Zigarette enthaltene Kapsel mit den Raucher-Entwöhnungsmitteln ("EZ-Quit" oder "Paipo") erzeugt, wobei Menthol einer der wesentlichen Bestandteile ist.

Es ist die Aufgabe der vorliegenden Erfindung, den Nachteilen der bekannten Entwöhnungsmethoden bzw. Entwöhnungsmittel abzuhelfen. Insbesondere soll ein einfaches und u.a. auch in der Natur vorkommendes Mittel vorgeschlagen werden, mit dem sich ein Raucher selbst oder unter Anleitung von Fachpersonal erfolgreich einem Nikotinentzug unterziehen kann und damit zum Nichtraucher wird.

Zur Lösung dieser Aufgabe wird die Verwendung eines Gemisches aus einem als Träger dienenden Öl mit einem Gehalt an mindestens 30 Volumen % Menthol zur Herstellung eines Arzneimittels für einen Nikotinentzug vorgeschlagen. Hierbei wird das Gemisch vorzugsweise auf die Papillen der Zunge bzw. der Mundschleimhaut aufgetragen. Zweckmäßige Ausgestaltungen der erfindungsgemäßen Verwendung gehen aus den Ansprüchen 2 bis 8 hervor.

Durch die hochkonzentrierte Zufuhr des Wirkstoffs Menthol wird hierbei ein aromatherapeutischer Effekt erzielt, der dazu führt, daß der Raucher kein Verlangen mehr auf den Konsum einer Zigarette bzw. eines sonstwie rauchbaren Artikels verspürt.

Es ist zwar bekannt, in Kräuterbonbons und dgl. eine geringe Konzentration an Menthol vorzusehen, die durch einen stetigen Speichelfluß noch stärker verdünnt wird, so daß allenfalls Mentholkonzentrationen von kleiner als 0,1% an den im Mund eines Menschen befindlichen Rezeptoren aufgetragen werden, diese haben jedoch in keinem Fall die überraschende Wirkung, die ein Gemisch aus einem vorzugsweise mindestens 40 Vol.% Menthol enthaltenden Öl erzielt.

Außerdem ist es bekannt, geringste Konzentrationen von Menthol in Alkohol als Trägersubstanz und zusätzlich in Wasser verdünnt z.B. für die Mundhygiene zu verwenden. Die hierbei empfohlenen Konzentrationen von weniger als 1/10 Promille sind jedoch ebenfalls nicht dazu geeignet, den gemäß der vorliegenden Erfindung überraschenderweise zu erzielenden aromatherapeutischen Effekt zu erhalten.

Schließlich ist es bekannt, Zigaretten mit Menthol-Aromabestandteilen herzustellen, woraus unmittelbar erkennbar ist, daß der gemäß der vorliegenden Erfindung erzielbare Effekt im Stand der Technik nicht bekannt ist. Außerdem ist der Mentholanteil im Rauch einer derartigen Zigarette noch wesentlich geringer als bei den oben aufgeführten Produkten.

Bei starken Rauchern ist es möglich, daß der starke Reiz, der eine Wirkung auf die Psyche bzw. auf Reflexnerven im Gehirn oder Stammhirn entfaltet, wodurch keine Entzugserscheinungen entstehen bzw. diese vorhandenen Entzugserscheinungen überdeckt werden, erst ab 30 Vol.%, vorzugsweise ab 60 oder gar 70 Vol.% Menthol in einer Flüssigkeit auftritt. Dies hängt damit zusammen, daß die Rezeptoren von starken Rauchern abgestumpft sein können.

Als Restbestandteil des Gemisches, das mit dem Menthol zu 100 Vol.% ergänzt ist, kann ein Öl verwendet werden. Z.B. können pflanzliche Öle, wie Sojaöl oder Sonnenblumenöl, zum Einsatz gelangen. Der Restbestandteil kann auch teilweise oder vollständig durch ätherische Öle gebildet werden. Vorzugsweise wird das Menthol jedoch in seinem natürlichen Vorkommen in Pfefferminzöl zum Einsatz gelangen. Hierbei wird das verwendete Gemisch ca. 80 bis 95 Vol.% Pfefferminzöl mit einem Mindestanteil von 50 Vol.% Menthol enhalten.

Bei Verwendung von 30 bzw. 40 Vol.% natürlichem oder synthetisch hergestelltem Menthol können die restlichen 70 bzw. 60 Vol.% des Gemisches aus einem oder mehreren zusätzlichen Ölen oder ätherischen Ölen zusammengestellt sein.

Als ätherische Öle kommen z.B. Kiefernnadelöle, Eukalyptusöle und Neikenöle in Betracht, die zusätzlich eine entschlackende und reinigende Wirkung auf das Atmungssystem eines Rauchers entfalten können.

Es kann auch zweckmäßig sein, Zusätze von Alkohol in das erfindungsgemäße Gemisch beizumengen. Auf diese Weise können auch Bestandteile hinzugefügt werden, die zwar nicht öl-, dafür aber alkohollöslich sind. Diese Bestandteile werden dann einer Alkoholfraktion beigemengt, die dann zum Bestandteil des erfindungsgemäßen Gemisches gemacht wird. Die Alkoholfraktion sollte jedoch 50 Vol.% nicht überschreiten, sondern eher zu 10 Vol.% oder weniger tendieren.

Es bleibt jedoch festzuhalten, daß die Erfindung auf der Erkenntnis beruht, Menthol in hochkonzentrierter Form Schleimhautrezeptoren zuzuführen, wodurch ein auf das Nervenzentrum wirkender Reiz ausgelöst wird, der über Stunden hinweg anhalten kann.

Das hierfür verwendete Menthol kann z.B. durch Unterkühlung von Pfefferminzöl gewonnen werden. Bei der Unterkühlung von Pfefferminzöl flockt Menthol aus und läßt sich anschließend von dem Pfefferminzöl trennen. Allerdings läßt sich Menthol auch synthetisch herstellen, und es ist durchaus möglich, auch mit synthetischem Menthol den gewünschten aromatherapeutischen Effekt zu erzielen.

Die Mengen, in denen das Menthol enthaltende Aromatherapeutikum eingenommen wird, können sehr gering sein. Wichtig ist aber, daß an den Rezeptoren zumindest kurzzeitig hochkonzentriertes Menthol anliegt, um die Reizung und die damit verbundene Überlagerung der Nikotinentzugserscheinungen zu bewirken. So können bereits Bruchteile von Millilitern, z.B. 0,04 ml, den gewünschten Effekt erzielen. Jedoch sollte nur in Ausnahmefällen mehr als 0,1 bis 10 Milliliter der Menthol enthaltenden Flüssigkeit bzw. Substanz in Kontakt mit den Mundschleimhäuten gebracht werden. Es ist empfehlenswert, wenn die betreffenden Rezeptoren des Menschen auch kurzzeitig (bis zu ca. 1 Minute) mit dem erfindungsgemäßen Gemisch geflutet werden. Nach der Einwirkung kann die erfindungsgemäße Substanz ausgespuckt, aber auch hinuntergeschluckt werden. Dabei kann sehr konzentriertes Menthol (ab ca. 30 oder 40 Vol. %) auch auf ein Zuckerstück oder dgl. gegeben werden, um verabreicht zu werden.

Da es sehr auf die Nervenreizung ankommt, die durch die intensive Wirkung konzentrierten Menthols auf die betreffenden Rezeptoren erzielt wird, und ein Teil dieser Wirkung auch vom Geschmack des Menthols herrühren sollte, kann es auch sehr vorteilhaft sein, Geschmacksmusterverstärker, wie z.B. Salze oder Glutamate, hinzuzufügen.

## Patentansprüche

1. Verwendung eines Gemisches aus einem als Träger dienenden Öl mit einem Gehalt an mindestens 30 Volumen % Menthol zur Herstellung eines Arzneimittels zum Auftragen auf die Mundschleimhaut oder auf die Papillen der Zunge zur Behandlung des Nikotinentzugs.

2. Verwendung nach Anspruch 1, wobei mindestens 50 Volumen % Menthol enthalten sind.

3. Verwendung nach Anspruch 1, wobei mindestens 60 Volumen % Menthol enthalten sind.

4. Verwendung nach einem der Ansprüche 1 bis 3, wobei das Menthol enthaltende Ölgemisch zumindest teilweise ein Pfefferminzöl ist.

5. Verwendung nach einem der Ansprüche 1 bis 4, wobei mindestens 80 Volumen % des Gemisches Pfefferminzöl ist, welches einen Anteil von mindestens 50 Volumen % Menthol enthält.

6. Verwendung nach einem der Ansprüche 1 bis 5, wobei bis zu 60 Volumen % des Gemisches aus einem oder mehreren zusätzlichen Ölen oder ätherischen Ölen besteht.

7. Verwendung nach Anspruch 6, wobei als zusätzliches ätherisches Öl Kiefernnadelöl, Eukalyptusöl und/oder Nelkenöl einzeln oder in Mischungen verwendet wird.

8. Verwendung nach einem der Ansprüche 1 bis 6, wobei ein Anteil von Alkohol und/oder Geschmacksverstärkern mit verwendet wird.

## Claims

1. Use of a mixture of an oil, which serves as a carrier, with a content of at least 30 percent by volume of menthol for the production of a pharmaceutical for application to the mucous membrane of the oral cavity or to the papillae of the tongue for treatment of nicotine addiction.

2. Use according to claim 1, wherein at least 50 percent by volume of menthol are contained.

3. Use according to claim 1, wherein at least 60 percent by volume of menthol are contained.

4. Use according to one of claims 1 to 3, wherein the oil mixture containing menthol is at least in part a peppermint oil.

5. Use according to one of claims 1 to 4, wherein at least 80 percent by volume of the mixture is peppermint oil, which contains a proportion of at least 50 percent by volume of menthol.

6. Use according to one of claims 1 to 5, wherein up to 60 percent by volume of the mixture consists of one or more secondary oils or essential oils.

7. Use according to one of claims 1 to 6, wherein pine needle oil, eucalyptus oil and/or clove oil is used individually or in mixtures as secondary essential oil.

8. Use according to one of claims 1 to 6, wherein a proportion of alcohol and/or flavour enhancers is used therewith.

## Revendications

1. Utilisation d'un mélange constitué d'une huile ou essence servant de support ayant une teneur d'au moins 30 % en volume de menthol pour la préparation d'un médicament à appliquer sur les muqueuses buccales ou les papilles de la langue pour le sevrage nicotinique.

2. Utilisation selon la revendication 1, contenant au moins 50 % en volume de menthol.

3. Utilisation selon la revendication 1, contenant au moins 60 % en volume de menthol.

4. Utilisation selon l'une des revendications 1 à 3, le mélange d'huile contenant le menthol étant au moins en partie une essence de menthe poivrée.

5. Utilisation selon l'une des revendications 1 à 4, dans laquelle au moins 80 % en volume du mélange est de l'essence de menthe poivrée qui contient une proportion d'au moins 50 % en volume de menthol.

6. Utilisation selon l'une des revendications 1 à 5, dans laquelle jusqu'à 60 % en volume du mélange étant constitué d'une ou de plusieurs essences supplémentaires ou d'essences d'éther.

7. Utilisation selon la revendication 6, dans laquelle on utilise en tant qu'essence d'éther supplémentaire, de l'essence d'aiguilles de pin, de l'essence d'eucalyptus et/ou de l'essence de girofle individuellement ou en mélange.

8. Utilisation selon l'une des revendications 1 à 6, une portion d'alcool et/ou d'agent renforçant le goût étant également utilisé.
